Europäisches Patentamt

(19) European Patent Office

Office européen des brevets

(11) Publication number: **0 103 646**
**B1**

## (12) EUROPEAN PATENT SPECIFICATION

(45) Date of publication of patent specification: **25.10.89**

(21) Application number: **83900851.3**

(22) Date of filing: **14.03.83**

(86) International application number:
**PCT/JP83/00077**

(87) International publication number:
**WO 83/03258 29.09.83 Gazette 83/23**

(51) Int. Cl.⁴: **C 12 N 1/16, C 12 N 5/00,**
**C 12 G 1/00**

(54) **NOVEL KILLER YEAST, PROCESS FOR ITS PRODUCTION, AND APPLICATION THEREOF.**

(30) Priority: **15.03.82 JP 41380/82**
**15.03.82 JP 41381/82**

(43) Date of publication of application:
**28.03.84 Bulletin 84/13**

(45) Publication of the grant of the patent:
**25.10.89 Bulletin 89/43**

(84) Designated Contracting States:
**AT BE CH DE FR GB LI LU NL SE**

(56) References cited:
**JP-A-56 005 088**
**JP-B-57 002 307**

**JOURNAL OF INST. OF BREWING, vol. 87, no.
5, September, October 1981, pages 292-295,
London, GB; T.W. YOUNG: "The genetic
manipulation of killer character into brewing
yeast"**

**CHEMICAL ABSTRACTS, vol. 92, no. 11, March
17, 1980, page 452, ref. no. 92656u, Columbus,
Ohio, US; R.B. WICKNER et al.: "Breeding of
killer yeasts for sake brewing by cytoduction";
& J. FERMENT. TECHNOL. 1979, 57(6), 483-7**

(73) Proprietor: **Suntory Limited**
**1-40 Doujimahama 2-chome Kita-ku
Osaka-shi Osaka-fu 530 (JP)**

(72) Inventor: **OHSHIMA, Takehiro
3-4, 17-10-383 Betsushohonmachi
Takatsuki-shi Osaka 569 (JP)**
Inventor: **TOUJOU, Kazumoto D5-203, 4
Shinsenrihigashimachi 2-chome
Toyonaka-shi Osaka 565 (JP)**
Inventor: **MIYAJIMA, Keiji
17-4, Onokosei 2-chome Shigacho
Shiga-gun Shiga 520-05 (JP)**

(74) Representative: **Hansen, Bernd, Dr.rer.nat. et al
Hoffmann, Eitle & Partner Patentanwälte
Arabellastrasse 4 Postfach 81 04 20
D-8000 München 81 (DE)**

(56) References cited:
**J.Inst.Brew., vol. 87 (1981), Sept.-Oct., pp.
292-295**

**J.Ferm.Technol., vol. 57 (6) (1977), pp. 483-487**

EP 0 103 646 B1

## Description

Technical Field

The present invention relates to a novel killer yeast, a process for its construction, and its use in wine making. More particularly, the invention relates to the construction of a useful yeast of homothallic strain into which a killer factor has been introduced, as well as wine making using said yeast.

Background Art

One of the problems that always bother producers of alcoholic beverages is contamination by unwanted microorganisms coming from the starting materials, the container and the atmosphere. In particular, differentiation between wild yeasts and useful yeasts is difficult to make by the environment, and the addition of a pure culture of useful strains as seeds does not eliminate the chance of contamination by wild yeasts, and this presents a great problem in quality control for the cellar master. For example, in wine making, 50 to 100 ppm of sulfites are added to the grape must in order to inhibit the growth of harmful wild yeasts and other microorganisms originating from the grape skins. The cellar master expects the sulfites to inhibit the growth of wild microorganisms while the useful yeast is growing. However, the sulfites added are not completely effective against the growth of undesired microorganisms. They are effective against "film-forming" yeasts of *Hansenula* and *Pichia*, but not against sulfite-resistant strains of *Saccharomyces,* which survive in the fermenting must and may contaminate the wine under aging of bottled wine. Pseudo-film forming yeast of *Saccharomyces bayanus* are particularly resistant to sulfites and they also have resistance to alcohols. If some of these yeasts grow in the must or wine, they either give an unpleasant taste or flavour or start another fermentation which may lead to burst bottles. One common practice in wine making is to recover the useful yeast from the fermenting must and use it in the next cycle of fermentation. In this case, if ordinary wine yeasts are used, a mixture of the useful yeast and the undesired yeast of *Saccharomyces* having high resistance to sulfites and alcohol is formed in the later stage of fermentation. Because of the high resistance to alcohol of this contaminating yeast, repeated use of the recovered yeast results in the increased proportion of the contaminating yeast. Obviously, the use of such recovered yeast as seed is not recommendable from a viewpoint of the control over microorganisms in the winery.

Methods have been proposed for producing a brewing yeast into which a killer factor has been introduced. According to these methods, the killer yeast is constructed by either (1) the backcross method after mating with a killer yeast (see Japanese publication no. 2307/82) or (2) the rate mating method between a brewing yeast and killer yeast (see Young, J. Inst. Brew. 1981, Vol. 87, pp 292—295). However, the first method involves complicated procedures, needs many days for breeding the desired yeast and has limited applicability to haploid yeast which usually lacks commercial feasibility. In the second method, an appropriate selection marker in a parent strain is necessary for the screening of fusion cells since the frequency of appearance of the killer brewing yeast is low. Furthermore, these methods are difficult to use in order to restore completely the genetic traits governed by the nucleic genes possessed by the useful parent strain.

Therefore, the principal object of the present invention is to provide a process for constructing a killer yeast which is free from the defects of the conventional technique.

Disclosure of the Invention

For constructing the desired killer yeast according to the present invention, it is essential that a completely homothallic yeast (see Reference 1 in the Bibliography at the end of this specification) be used as the useful yeast and that a karyogamy deficient mutant be used as the strain carrying a killer factor. A killer factor cannot be introduced into a completely homothallic strain by the conventional method of mating. However, the present inventors noted that the spores of a completely homothallic yeast are of either a- or α-mating type and that after two cell divisions, conversion of the mating type is caused, and that the two types of cells mate each other to form a diploid. Based on these observations, the inventors proceeded with the researches and finally accomplished the present invention. The invention comprises the yeast strain SAV—301 (FERM BP—261) of *Saccharomyces cerevisiae*, a method for constructing a killer yeast according to claims 2 and 3 as well as a method of wine making according to claims 4 to 6.

According to the present invention, the spores of a useful homothallic yeast are mated with a killer yeast defective in nuclear fusion (karyogamy deficient mutant) before the homothallic spores undergo two cell divisions. By doing this, diploid or polyploid cells of the desired useful yeast having the killer factor and the nucleic genome only derived from the useful parent yeast can be constructed. Any diploid or polyploid yeast is suitable as the useful parent yeast if it has the homothallic mechanism. A suitable example is ATCC 32747. All karyogamy deficient mutants, such as JCXIP 24 (see Reference 2) may be used as the killer yeast. Adding non-sensitivity or sensitivity to antiobiotics to the killer yeast as a selective marker is preferred for facilitating the selection of the desired useful yeast to be bred.

As shown in Example 1, the present invention successfully constructed a killer wine yeast by mating, through cytoduction, SY—5 yeast with a homothallic wine yeast SW—001 which has a low tendency to settle and a high suspensibility, as well as high survival ratio in the later stage of fermentation.

By using the so-constructed killer yeast (*Saccharomyces cerevisiae* SAV—301) as a wine yeast, contamination of the fermenting must by undesired yeasts could be suppressed and wine with high quality could be made. By increasing the lethality of undesired microorganisms by the useful killer yeast, the present inventors recovered an adequate quantity of the yeast suspended in the supernatant of the fermenting must while avoiding the use of the settling yeast severely contaminated by pectin, proteins, tannin and coloring matter originating from the grape skins. As a result, not only the number of living cells in the recovered yeast but also the volume of the useful killer yeast could be increased to such an extent that the growth of harmful yeasts was effectively prevented. This method enabled continuous recovery of the desired yeast and simple preparation of a seed yeast, and hence, wine of consistent quality could be made. The present invention provides the first commercial method for making quality wine by repeatedly using the recovered useful yeast in fermentation of the grape must.

Wine making with a useful wine yeast having a killer factor introduced thereinto is already known (Japanese Patent Publication No. 2307/82). However, the killer yeast constructed by this prior art method has almost the same lethality after grape must fermentation as the ordinary wine yeast, and therefore, it is not highly recommended to recover this killer yeast and use it in repeated fermentation.

The advantages of the present invention are hereunder described in greater detail by reference to nonlimiting working examples. Although the following Examples are illustrated by using specific strains of SW001 and SY—5, any equivalent strains can be used. That is, any diploid or polyploid yeast may be used in this invention as the useful parent yeast provided that it has the homothallic mechanism. In addition, any karyogamy deficient mutant having the killer factor such as JXCIP may be used as the killer donor yeast.

## Example 1

Construction of Killer Wine Yeast

Diploid wine yeast *Saccharomyces cerevisiae* SW—001 (2 ml) was cultured on a YPD medium containing 1% yeast extract, 2% polypeptone and 2% glucose by leaving the medium to stand at 30°C. The culture was then placed on a spore-forming medium (0.5% potassium acetate and 2% agar) and cultured at 30°C for 24 hours to form spores.

Killer yeast *Saccharomyces cerevisiae* SY—5 (a leu 1 Kar 1—1[K+][Ery$^r$]) was cultured on a YPD medium for 24 hours. The spores of SW—001 strain and SY—5 cells were placed on a small YPD agar medium and under observation by a microscope, the two groups of cells were brought into contact with each other by manipulation of a thin glass rod (for the method of spore to cell mating, see Reference 3). After confirming that the spores fused to the cells under microscope, the small YPD agar medium was transferred to a YPD agar plate medium and cultured at 30°C for 3 days until colonies formed. Cells from the colonies were picked up and cultured on a minimum nutrient medium (product of Difco, containing 3% glycerol and 0.67% yeast nitrogen base) combined with 100 μg/ml of erythromycin. Two independent matings were conducted on a 3% glycerol-erythromycin minimum medium, and 5 clones were collected from each cross, and subjected to tetrad analysis to see the killer-behaviour of the clones. The result showed that killer yeast, SAV—301, having the desired SW—001 nucleic gene was constructed. The strain has been deposited with the Fermentation Research Institute, the Agency of Industrial Science & Technology, under FERM BP—261. The strains used in the matings and their microbiological data are identified in Table 1. Further analysis showed that the killer yeast (SAV—301) carried the same cytoplasmic M dsRNA and L dsRNA plasmids concerning expression of killer factor as those of SY—5 in their molecular weights. The killer spectrum of SAV—301 was identical with that of SY—5, as shown in Table 2. The mycological properties of SAV—301 (see Reference 4) were entirely the same as those of SW—001. The sugar fermentation data of these two strains is given in Table 3.

## Table 1

| Strain | SW-001 | SAV-301 | SY-5 |
|---|---|---|---|
| Nucleus | SW-001 | SW-001 | SY-5 |
| Ploidy | 2n | 2n | 1n |
| Killer | - | + | + |
| Erythromycin resistance | S | R | R |
| Tetrad analysis<br>  leucine-requirement<br>  homo : hetero | $4^+$ : $0^-$<br>4 : 0 | $4^+$ : $0^-$<br>4 : 0 | -<br>- |

EP 0 103 646 B1

Table 2

|  | | SW-001 | SAV-301 | SY-5 |
|---|---|---|---|---|
|  | a | − | + | + |
|  | b | − | + | + |
|  | c | − | + | + |
|  | d | − | + | + |
|  | e | − | + | + |
|  | f | − | + | + |
|  | g | − | + | + |
|  | h | − | + | + |
|  | i | − | + | + |
|  | j | − | + | + |
|  | k | − | + | + |
|  | l | − | + | + |

a) *Saccharomyces bayanus* possessed by Suntory, Ltd.
b) *Saccharomyces beticus* possessed by Suntory, Ltd.
c) *Saccharomyces oviformis* possessed by Suntory, Ltd.
d) *Saccharomyces fermentati* possessed by Suntory, Ltd.
e) *Saccharomyces lactis* possessed by Suntory, Ltd.
f) *Saccharomyces cerevisiae*, Kyokai No. 7
g) *Saccharomyces willianus* IFO-248
h) *Saccharomyces cerevisiae var. eliipsoidenus* IFO-251
i) *Saccharomyces oviformis* IFO-262
j) *Saccharomyces cerevisiae* IFO-304
k) *Saccharomyces diastaticus* IFO-1015
l) *Saccharomyces diastaticus* IFO-1046

Table 3

|  | SAV-301 | SW-001 |
|---|---|---|
| glucose | + | + |
| galactose | + | + |
| sucrose | + | + |
| maltose | + | + |
| lactose | − | − |
| raffinose | + | + |
| melibiose | − | − |

+ fermented, − not fermented

Example 2
Growth Profile of Contaminants in Fermenting Must and Quality of Wine
    Bunches of grapes (variety: Koshu, 20 kg) were crushed, stripped of their stems and immediately pressed to extract juice. The juice was supplemented with 100 ppm of potassium metabisulfite chaptilized to a sugar content of 20.2%, centrifuged and aseptically passed through an EK filter (product of Seitz-Werke GmbH). The filtered juice was put into sterilized fermentation vats. To one vat, pure cultures of killer yeast

4

SAV—301 and contaminating yeast *Saccharomyces bayanus* FB—1 were added (method A) and the juice was fermented at 20°C. To the other vat, pure cultures of wine yeast *Saccharomyces cerevisiae* SW—001 and contaminant FB—1 (method B) were added and the juice was also fermented at 20°C. In method A, the juice contained $2 \times 10^6$ cells/ml of SAV—301 and $2 \times 10^5$ cells/ml of FB—1. In method B, the juice contained $2 \times 10^6$ cells/ml of SW—001 and $2 \times 10^5$ cells/ml of FB—1.

On the 7th day of the fermentation, the number of living cells in each vat was counted. All the yeast cells present in the juice fermented by method A were those of the killer yeast, wherein 29.8% of the yeast cells present in the juice fermented by method B originated from contaminant yeast FB—1. The killer yeast was distinguished from the contaminant yeast by the resistance to erythromycin (100 μg/ml) of the colonies formed on a nutrient agar medium (the killer yeast was erythromycin-resistant but the contaminant yeast was not).

To each sample of the fermented juice, 300 ppm of potassium metabisulfite was added, and the amounts of the free form and bound form of the sulfite were measured. The wine samples were also subjected to an organoleptic test by 11 panelists. These results are shown in Tables 4 and 5.

## Table 4

| Parameter \ Method | A | B |
|---|---|---|
| free sulfite (mg/1,000 ml) | 19 | 5 |
| bound sulfite (mg/1,000 ml) | 139 | 149 |
| total sulfite (mg/1,000 ml) | 158 | 154 |

The determination of free and bound sulfites was made in accordance with the Rankine method (1962) (see Reference 5).

## Table 5

| | Method A | Method B | $t_o$ | Significant difference |
|---|---|---|---|---|
| Color | 1.5 | 1.5 | 0.00 | |
| Aroma | 2.7 | 1.8 | 3.38 | * * |
| Flavor | 10.1 | 8.9 | 3.66 | * * |
| Total | 14.3 | 12.2 | 4.51 | * * |

In the organoleptic test, the full score was 18 points consisting of 0 to 2 points for color, 0 to 4 points for aroma and 0 to 12 points for flavor.

Table 4 shows that the juice fermented by method A contained a greater amount of the free sulfite than the juice fermented by method B. It is therefore concluded that method A would be more effective than method B in preventing oxidation and contamination by undesired microorganisms. In Table 5, ** indicates the presence of a significant difference at a probability of 0.01. The t distribution table shows that the value for two-tailed $t_{0.01}$ was 3.106 for 10 df. The $t_o$ values for the tests of aroma, flavour and overall evaluation were greater than 3.106. Therefore, it was concluded that there was a significant difference between the killer yeast of the present invention and the conventional wine yeast.

As will be apparent from the above data, the wine made from the juice fermented in the presence of killer yeast SAV—301 to inhibit the contaminating yeast was more prone to form free sulfite and smelled less of the unpleasant aldehyde.

## Example 3

Effective of Killer Yeast Against Re-Fermentation

To fresh Delaware grape juice (sugar content; 18.0%, acidity: 0.89%), glucose was added to increase the sugar content of 19.9%. The juice was fined by centrifuge and aseptically passed through a membrane filter (Millipore Limited). Five liter portions of the juice were transferred into two fermentation vats sterilized by dry heat.

To one vat, a pure culture of killer yeast SAV—301 was added in an amount of 5% of the volume of the juice, and to the other vat, a pure culture of conventional wine yeast SW—001 was added in the same amount. The respective must samples were fermented at 20°C.

When the sugar content of the juice decreased to about 1.3%, the juice was cooled to stop the fermentation. After racking, filtration and fining, 70 ppm of sulfite was added to the juice, which was aseptically passed through a membrane filter (Millipore Limited) and transferred into two fermentation vats sterilized by dry heat.

Wine was made from the must that had been fermented in the presence of killer yeast SAV—301, and $2 \times 10^5$ cells/100 ml of a pure culture of contaminating yeast *Saccharomyces bayanus* FB—1 were added to one sample of the wine, whereas $2 \times 10^6$ cells/100 ml of the same yeast were added to another sample. Wine was also made from the must that had been fermented in the presence of conventional wine yeast SW—001. To one sample, $2 \times 10^5$ cells/100 ml of a pure culture of FB—1 were added, and to another sample, $2 \times 10^6$ cells/100 ml of the same yeast were added. The four wine samples were left to stand at 20°C to see if another fermentation would occur. The bacterial growth was observed by checking the turbidity of the wine with a spectrophotometer ("Spectronics 20A" of Shimazu-Bausch & Lomb) at 660 nm. The results are shown in Table 6, from which one can see that the killer yeast of the present invention retarded the microbial growth more effectively than the conventional wine yeast and hence was more effective against re-fermentation.

## Table 6

| Conditions | Days of culture | | | |
|---|---|---|---|---|
| | 2 | 5 | 7 | 10 |
| a | 0.02 | 0.122 | 0.395 | 0.733 |
| b | 0.02 | 0.230 | 0.469 | 0.809 |
| c | 0.01 | 0.02 | 0.151 | 0.767 |
| d | 0.01 | 0.02 | 0.282 | 0.864 |

The culture conditions a to d were as follows:

| | Cell count | Sample |
|---|---|---|
| a | $2 \times 10^6$ cells/100 ml | wine made from the must fermented in the presence of killer yeast SAV-301 |
| b | $2 \times 10^6$ cells/100 ml | control |
| c | $2 \times 10^5$ cells/100 ml | wine made from the must fermented in the presence of killer yeast SAV-301 |
| d | $2 \times 10^5$ cells/100 ml | control |

## Example 4

Effect of Killer Yeast on Continuous Fermentation

Bunches of grapes (variety: Koshu, 2 kg) were crushed, stripped of their stems and immediately pressed to extract juice. The juice was supplemented with 100 ppm of potassium metabisulfite, chaptalised to a sugar content of 21.6%, centrifuged to remove any turbidity, and aseptically passed through an EK filter (product of Seitz-Werke GmbH).

# EP 0 103 646 B1

The filtered juice was put into sterilized fermentation vats. To one vat, pure cultures of killer yeast SAV—301 and contaminating yeast *Saccharomyces bayanus* FB—1 were added (method A), and the juice was fermented at 20°C. To the other vat, pure cultures of wine yeast SW—001 and contaminant FB—1 were added (method B), and the juice was also fermented at 20°C. In method A, the must contained $4 \times 10^6$ cells/ml of SAV—301 and $4 \times 10^5$ cells/ml of FB—1. In method B, the must contained $4 \times 10^6$ cells/ml of SW—001 and $4 \times 10^5$ cells/ml of FB—1.

When the sugar content of each must became about 1%, the yeasts were recovered from the must. Grape juice was prepared in the same manner as used above, and $4 \times 10^6$ cells/ml of the respective recovered yeasts and $4 \times 10^5$ cells/ml of a pure culture of FB—1 were added to the juice, and the must was fermented at 20°C. The same procedure was used in another cycle of fermentation. The proportions of killer yeast SAV—301 and contaminant yeast FB—1 that survived in the respective stages of fermentation are shown in Table 7. Almost all of the living cells in the must that underwent the three cycles of fermentations according to method A originated from the killer yeast, and the growth of the contaminating yeast was negligible. On the other hand, 80.5% of the living cells in the must fermented according to method B originated from the contaminant yeast.

The grape juice is usually fermented unaseptically. The above results show that even in this case, the cells of a recovered yeast containing the killer yeast of the present invention can be continuously used as seeds in fermentation of the grape must.

## Table 7

| Method | A | | B | |
|---|---|---|---|---|
| yeast | killer yeast SAV-301 | contami- nating yeast FB-1 | wine yeast SW-001 | contami- nating yeast FB-1 |
| Immediately after the first addition of recovered yeast | 90.9% | 9.1% | 90.9% | 9.1% |
| Lethality | 5.0% | | 8.0% | |
| Immediately after the second addition | 90.8% | 9.2% | 70.2% | 29.8% |
| Lethality | 8.0% | | 15.0% | |
| Immediately after the third addition | 99.9% | 0.1% | 19.5% | 80.5% |
| Lethality | 10.0% | | 20.0% | |

## Example 5

Continuous Fermentation with Killer Yeast in Unsterilized Juice

Bunches of grapes (variety: Koshu, 150 kg) were crushed, stripped of their stems and immediately pressed to extract juice. The juice was supplemented with 100 ppm of sulfite, chaptalized to a sugar content of 22%, centrifuged to obtain a clear must.

A pure culture of killer yeast SAV—301 was added to the must in an amount of 4% on the basis of the volume of the must. The must was fermented at 20°C. When the sugar content was reduced to about 1%, the supernatant was centrifuged to recover the yeast. Another sample of the must was prepared in the same manner as above, and $4 \times 10^6$ cells/ml of the recovered yeast were added to this must which was then fermented at 20°C. The same procedure was repeated five times and the yeast was recovered. No typical contaminant yeast *Saccharomyces bayanus* was detected in the recovered yeast.

7

## EP 0 103 646 B1

*Bibliography:*
(1) Takano, I., Oshima, Y.: Genetics, 65, 421 (1970)
(2) J. Conde and G. R. Fink: Proc. Natl. Acad. Sci. USA, 73, 3651 (1976)
(3) Takano, I., Oshima, Y.: Genetics, *57*: 875 (1967)
(4) Lodder: The Yeasts, 2nd edd. (1970)
(5) Rankine: Aust., Wine, Brew. and Spir. Rev. *80* (5) 14

### Claims

1. Yeast strain SAV—301 (FERM BP—261) of Saccharomyces cerevisiae.

2. A method of constructing a killer yeast comprising fusing a karyogamy deficient mutant yeast harboring killer plasmids to the spore produced by meiosis of a diploid or polyploid homothallic yeast before the homothallic spores undergo two cell divisions and selecting the desired cells from those cells which bud from the fused cells by subjecting to tetrad analysis to see the killer-behavior of the cells.

3. A method according to claim 2 wherein the homothallic yeast is a homothallic yeast used in the fermentation for making alcoholic beverages.

4. A method of making wine, characterized by using the killer yeast constructed by the method according to claim 2.

5. A method according to claim 4 wherein the killer yeast strain is SAV—301 (FERM BP—261).

6. A method according to claim 4 or 5 where the yeast that is recovered from the must after one or more cycles of fermentation is used as the seed yeast.

### Patantansprüche

1. Hefestamm SAV—301 (FERM BP—261) von Saccharomyces cerevisiae.

2. Verfahren zur Herstellung einer keimtötenden Hefe, wobei man eine karyogamiedefiziente mutante Hefe, die keimtötende Plasmide enthält, mit einer Spore fusioniert, die durch Meiose einer diploiden oder polyploiden homothallischen Hefe erzeugt wurde, bevor die homothallischen Sporen zwei Zellteilungen eingehen, und wobei man die gewünschten Zellen aus denjenigen Zellen, die aus den fusionierten Zellen keimen, durch Tetra-Analyse auswählt, um das keimtötende Verhalten der Zellen festzustellen.

3. Verfahren gemäß Anspruch 2, dadurch gekennzeichnet, daß die homothallische Hefe eine homothallische Hefe ist, die in der Gärung zur Herstellung alkoholischer Getränke verwendet wird.

4. Verfahren zur Herstellung von Wein, dadurch gekennzeichnet, daß man die keimtötende Hefe, hergestellt durch das Verfahren gemäß Anspruch 2, verwendet.

5. Verfahren gemäß Anspruch 4, dadurch gekennzeichnet, daß der keimtötende Hefestamm SAV—301 (FERM BP—261) ist.

6. Verfahren gemäß Anspruch 4 oder 5, dadurch gekennzeichnet, daß die Hefe, die aus dem Most nach einem oder mehreren Gärungszyklen wiedergewonnen wird, als Keimhefe verwendet wird.

### Revendications

1. Souche de levure SAV—301 (FERM BP—261) de *Saccharomyces cerevisiae*.

2. Procédé pour élaborer une levure tueuse, consistant à faire fusionner une levure mutante à déficience caryogamique recélant des plasmides tueurs avec la spore produite par méiose d'une levure homothallique diploïde avant que les spores homothalliques ne subissent deux divisions cellulaires et à sélectionner les cellules désirées parmi les cellules qui sortent par bourgeonnement des cellules fusionnées en les soumettant à une analyse des tétrades pour examiner le comportement tueur des cellules.

3. Procédé selon la revendication 2, dans lequel la levure homothallique est une levure homothallique utilisée dans la fermentation pour la fabrication de boissons alcoolisées.

4. Procédé de vinification, caractérisé par l'utilisation de la levure tueuse élaborée par le procédé selon la revendication 2.

5. Procédé selon la revendication 4, dans lequel la souche de levure tueuse est SAV—301 (FERM BP—261).

6. Procédé selon la revendication 4 ou 5, dans lequel la levure qui est récupérée en étant retirée du moût après un ou plusieurs cycles de fermentation est utilisée comme levure de semence.